# EUROPÄISCHE PATENTSCHRIFT

(11) **EP 3 628 371 B1**
(45) Veröffentlichungstag und Bekanntmachung des Hinweises auf die Patenterteilung: **24.08.2022**
(21) Anmeldenummer: 19198175.2
(22) Anmeldetag: 19.09.2019
(51) Int. Cl.: A61N 5/10

(54) **APPLIKATOR FÜR DIE INTRAOPERATIVE STRAHLENTHERAPIE**
APPLICATOR FOR INTRAOPERATIVE RADIATION THERAPY
APPLICATEUR POUR LA RADIOTHÉRAPIE INTRAOPÉRATIVE

(30) Priorität: 28.09.2018 DE 102018216760
(43) Veröffentlichungstag der Anmeldung: 01.04.2020
(73) Patentinhaber: Carl Zeiss Meditec AG, 07745 Jena (DE)
(72) Erfinder: BAUMSTARK, Helge, 73450 Neresheim (DE)
(74) Vertreter: Carl Zeiss AG - Patentabteilung

(56) Entgegenhaltungen:
- WO-A1-2009/108909
- WO-A1-2015/158397
- WO-A2-2006/047112
- WO-A2-2007/108854
- WO-A2-2009/111060
- WO-A2-2010/060097
- US-A1- 2008 009 730

## Beschreibung

Die Erfindung betrifft ein Applikator-System mit einem Applikator für die intraoperative Strahlentherapie mit niederenergetischer Röntgenstrahlung und einer elastischen Sterilhülle. Bei der intraoperativen Strahlentherapie kommen Applikatoren zum Einsatz, die an einer Bestrahlungsvorrichtung angeordnet sind. Die Bestrahlungsvorrichtung umfasst eine Röntgenquelle mit einer Anschlussstelle für einen Applikator. An der Anschlussstelle ist ein langgestrecktes Rohr angeordnet, dessen distales Ende die Röntgenquelle bildet. Ein Elektronenstrahl trifft dabei auf ein schweres Metall-Target, vorzugsweise ein Gold-Target, das am distalen Ende des Rohres angeordnet ist, so dass beim Auftreffen des Elektronenstrahls auf das schwere Metall Röntgenstrahlung erzeugt wird.

An der Anschlussstelle der Bestrahlungsvorrichtung ist ein hülsenförmiger Applikator angeordnet, in dessen inneren Bereich das Rohr mit der Röntgenquelle angeordnet ist. Der Applikator ist aus einem strahlungsdurchlässigen Material hergestellt und ist in seiner äußeren Geometrie an eine Körperöffnung oder eine durch eine Operation entstandene Form einer Körperöffnung angepasst. Der Applikator kann abhängig von der Art oder der Größe einer Körperöffnung gewählt werden. Abhängig vom Anwendungsfall können Applikatoren in verschiedenen Größen und Formen zum Einsatz kommen.

Der Applikator und die innerhalb des Applikators angeordnete Röntgenquelle können in die Körperöffnung eines Patienten eingeführt werden. Die Röntgenquelle kann dort Röntgenstrahlung für therapeutische Zwecke, beispielsweise zur Bestrahlung eines Tumors, erzeugen. Da der Applikator invasiv, d. h. in den Gewebebereich eines Körpers eingebracht wird, reicht niederenergetische Strahlung zur Behandlung des Körpergewebes aus. Als niederenergetische Röntgenstrahlung in dieser Anmeldung wird Strahlung definiert, die durch Auftreffen eines Elektronenstrahl s mit einer kinetischen Energie von maximal 100 keV auf das schwere Metall-Target, vorzugweise eine Gold-Target, erzeugt wird.

Durch den Applikator wird das Körpergewebe in einem Abstand von der Röntgenquelle gehalten, um eine gleichmäßige Bestrahlung zu bewirkten und/oder um ein lokal stark überhöhte Röntgendosis in der direkten Umgebung der Röntgenquelle zu vermeiden. Applikatoren kommen bei einer Anwendung in Kontakt mit Körpergewebe. Deshalb müssen die Applikatoren nach einer Anwendung entsorgt oder sterilisiert werden. Nach einer limitierten Anzahl von Resterilisationszyklen ist der Applikator schließlich nicht mehr verwendbar und muss entsorgt werden.

Aus der WO 2006/047112 A2 ist ein Applikator bekannt, der einen nicht deformierbaren Körper mit einem Kopf aufweist, der zur Positionierung in Körperhohlräumen ausgebildet ist, die durch die Entfernung eines Tumors entstanden sind.

Aus der WO 2015/158397 A1 ist eine Abdeckung für den Handgriff einer medizinischen Lichtquelle bekannt.

Die WO 2010/060097 A2 offenbart eine Vorrichtung zur Behandlung weichen Gewebes, die eine Lichtquelle und wenigstens zwei optische Anordnungen aufweist.

Die WO 2007/108854 A2 offenbart ein Applikator-System zur Anwendung akustischer Druckwellen.

Die US 2008/009730 A1 offenbart ein Verfahren zum Anbringen weichen Gewebes an eine Knochenmasse.

Aus der WO 2009/111 060 A2 ist ein Applikator mit einem Handgriff und einem Spitzenabschnitt bekannt, wobei der Spitzenabschnitt eine äußere Haut und einen Kern innerhalb der äußeren Haut aufweist.

Aus der WO 2009/108909 A1 ist ein Applikator bekannt, der einen länglichen Körper aus einem Schaummaterial aufweist.

Aufgabe der Erfindung ist es, einen verbesserten Applikator für die intraoperative Strahlentherapiebereitzustellen, mit dem die Anzahl der Anwendungen vergrößert werden kann.

Die Aufgabe wird durch eine Vorrichtung mit den Merkmalen des unabhängigen Anspruchs 1 gelöst. Die Aufgabe wird ferner durch ein Verfahren gemäß Anspruch 11 gelöst. Vorteilhafte Weiterbildungen der Erfindung sind in den Unteransprüchen beschrieben. Erfindungsgemäß umfasst das Applikator-System einen Applikator für die intraoperative Strahlentherapie mit niederenergetischer Röntgenstrahlung und eine elastische Sterilhülle, die an der Außenoberfläche des Applikators anordenbar ist. Der Applikator umfasst einen Applikatorkörper, eine Außenoberfläche mit einer Umfangsaußenfläche und einem distalen Ende, eine Aufnahmevorrichtung an einem proximalen Ende, mit der der Applikator an einer Röntgenbestrahlungsvorrichtung befestigbar ist, und eine innere Aussparung mit einer Öffnung an dem proximalen Ende, in die eine Röntgenstrahlungsquelle einführbar ist.

Der Applikator weist an seiner Außenoberfläche eine feste poröse Struktur auf, wobei die feste poröse Struktur eine luftdurchlässige zusammenhängende Kanalstruktur bildet, die luftleitend mit dem proximalen Ende des Applikators verbunden ist. Insbesondere verleiht die feste poröse Struktur der Außenoberfläche des Applikators eine starre Form. Die damit einhergehende genau definierte Bestrahlungsgeometrie ermöglicht eine sehr präzise Vorhersage und Vorgabe der auf das zu behandelnde Körpergewebe einwirkenden Strahlendosis.

Ein Applikator ist ein formgebendes Applikationsteil für die intraoperative Strahlentherapie mit niederenergetischer Röntgenstrahlung.

Die Außenoberfläche ist definiert als die Oberfläche des Applikatorkörpers an seinem Umfangsbereich und an seinem distalen Ende. Ein proximales Ende des Applikatorkörpers ist zur Befestigung an einer Röntgenbestrahlungsvorrichtung ausgebildet und hat aus diesem Grund eine Form, die für eine definierte Positionierung und Fixierung an der Röntgenbestrahlungsvorrichtung ausgebildet ist. Wenn der Applikator an der Röntgenbestrahlungsvorrichtung befestigt ist, liegt ein Bereich des proximalen Endes an der Röntgenbestrahlungsvorrichtung an. Dieser anliegende Bereich wird nicht als Außenoberfläche betrachtet.

Der Applikator hat eine innere Aussparung oder einen Hohlraum. Die Aussparung oder der Hohlraum hat eine Öffnung an dem proximalen Ende. Der Applikator ist hülsenförmig. Somit ist eine Röntgenstrahlungsquelle durch die Öffnung an dem proximalen Ende des Applikators in die Aussparung oder den Hohlraum des Applikators einführbar. Die Röntgenstrahlungsquelle umfasst ein Rohr, an dessen distalem Ende eine Strahlungsquelle gebildet ist. Die Strahlungsquelle ist somit innerhalb des Applikators angeordnet.

Der Applikator weist an seiner Außenoberfläche eine feste poröse Struktur auf, wobei die feste poröse Struktur eine luftdurchlässige zusammenhängende Kanalstruktur bildet, die luftleitend mit dem proximalen Ende des Applikators verbunden ist, so dass Luft von der gesamten Außenoberfläche zu dem proximalen Ende des Applikators leitbar ist. Ein Luftstrom kann dabei in beide Richtungen erfolgen. Es kann Luft aus der Kanalstruktur abgesaugt oder eingeblasen werden.

Unter einer porösen Struktur wird in dieser Anmeldung eine feste aber luftdurchlässige Materialstruktur verstanden, die mit Poren, Löchern, Kanälen oder Hohlräumen versehen ist. Die Poren, Löcher, Kanäle oder Hohlräume sind luftdurchlässig miteinander verbunden, so dass ein verzweigtes System aus vielen Luftkanälen gebildet ist, die luftleitend mit dem proximalen Ende des Applikators verbunden sind. Damit kann ein Luftstrom von jedem Bereich der Außenoberfläche zu dem proximalen Ende Applikators gebildet werden. Der Begriff der Porosität wird als Geometrieeigenschaft definiert und stellte als Messgröße das Verhältnis eines Hohlraumvolumens zum Gesamtvolumen des als porös definierten Bereiches des Applikators dar. Es wird an dieser Stelle darauf verwiesen, dass ein möglicherweise umgangssprachliches Verständnis des Begriffes "porös", der eine instabile Materialeigenschaft beschreiben würde, hier nicht von dem Begriff "porös" umfasst ist.

Eine poröse Struktur beschreibt eine funktionelle luftleitende Struktur, die sich von der Außenoberfläche bis in einen Tiefenbereich hinein erstreckt. Die poröse Struktur ist somit eine funktionell definierte Struktur und unterscheidet sich somit von einer durch eine Fertigungsart zufällig resultierende Oberflächenrauheit. Die Poren, Löchern, Kanälen oder Hohlräume sind im Verhältnis zur Größe des Applikators sehr klein und haben keinen oder nur sehr geringen Einfluss auf die äußere Geometrie des Applikators.

Die poröse Oberflächenstruktur betrifft den gesamten Bereich der Außenoberfläche, wobei jedoch ein Bereich an dem proximalen Ende des Applikators eine Oberfläche aufweisen kann, die nicht porös ausgestaltet ist.

Der Applikator kann für eine Anwendung individuell angefertigt werden. Auch eine individuelle Herstellung für einen Patienten ist vorstellbar. Der Applikator kann eine beliebige Außenkontur aufweisen.

Durch die funktionelle poröse Oberflächenstruktur der Außenoberfläche des Applikators kann Luft angesaugt oder ausgeblasen werden. Dadurch kann durch den Applikator eine Kraft auf ein weiteres Element ausgeübt werden, das zwischen Applikator und einem Gewebebereich eingesetzt werden kann. Ein Beispiel für ein weiteres Element kann ein Formteil mit dünner Wandstärke oder eine Folie bilden.

Dadurch kann der Applikator für die intraoperative Strahlentherapie mit niederenergetischer Röntgenstrahlung für eine große Anzahl von Anwendungen bereitgestellt werden. Durch seine funktionelle Ausbildung kann der Applikator eine Saugkraft oder eine Druckkraft auf ein Element ausüben, dass mit einem Gewebebereich in Kontakt kommt und zwischen dem Applikator und dem Gewebe angeordnet ist. Der Applikator selber kommt somit nicht mehr in direkten Kontakt mit einem Gewebebereich. Damit können an den Applikator geringere Anforderungen an Biokompatibilität, Sterisierbarkeit und Temperaturbeständigkeit gestellt werden. Ein Applikator kann häufiger eingesetzt werden. Die Anzahl der Anwendungen, bzw. die Einsatzhäufigkeit, kann vergrößert werden.

In einer Ausgestaltung der Erfindung ist die feste poröse Struktur durch eine feine Kanalstruktur gebildet, die an der Außenoberfläche des Applikators angeordnet ist.

Eine Kanalstruktur kann direkt an der Oberfläche angeordnet werden. Damit kann eine luftleitende strukturierte Außenoberfläche nachträglich an einem Applikator aufgebracht werden. Unter einer feinen Kanalstruktur wird eine Kanalstruktur mit einer Kanalbreite bis zu 3 mm verstanden. Die Kanalbreite ist in einer Ausführungsform zu dem proximalen Ende hin vergrößert ausgebildet.

In einer Ausgestaltung der Erfindung sind innerhalb des Applikatorkörpers Kanäle angeordnet, die von dem proximalen Ende zu der luftdurchlässigen Außenoberfläche geführt sind, so dass die luftdurchlässige Außenoberfläche des Applikators luftleitend mit dem proximalen Ende verbunden ist.

Um größere Volumenströme bereitzustellen können Kanäle auch innerhalb des Applikatorkörpers vorgesehen sein. Diese Kanäle können beispielsweise durch Bohrungen in den Applikator eingebracht werden. Damit kann der Luftstrom optimiert werden und ein gleichmäßiger Luftstrom über die ganze Außenoberfläche erreicht werden.

In einer Ausführungsform der Erfindung erstreckt sich die feste poröse Struktur von der Außenoberfläche bis in eine Tiefe von 2 mm, bevorzugt bis eine Tiefe von 3 mm, weiter bevorzugt bis in eine Tiefe bis 5 mm, weiter bevorzugt bis in eine Tiefe von 10 mm.

Die Herstellung einer porösen Struktur kann teurer sein, als die Herstellung einer massiven Struktur. Deshalb kann ein Applikator, bei dem eine feste poröse Struktur nur bis in einen Tiefenbereich ausgebildet ist, kostengünstiger sein.

Der Applikator kann rotationssymmetrisch sein.

Ein rotationssymmetrischer Applikator kann kostengünstig als Drehteil hergestellt werden.

Der Applikator kann einteilig ausgeführt sein.

Eine einteilige Form ist kostengünstig, leicht zu handhaben und kann schnell und einfach an der Röntgenbestrahlungsvorrichtung angebracht werden.

Der Applikator kann mehrteilig ausgeführt sein.

Eine mehrteilige Form bietet die Möglichkeit, komplexere Formen eines Applikators auszubilden. Es ist vorstellbar, das einen Applikator in einem "Baukastensystem" aus zwei oder mehr kompatiblen Einzelelementen zusammensetzbar ist. Mehrteilige Applikatoren, die spezifische Formen bilden, können die Behandlungszeit in einem Gewebe durch optimierte Strahlungsverteilung reduzieren.

In einer Ausgestaltung der Erfindung weist der gesamte Applikatorkörper eine feste poröse Struktur aufweist.

Damit kann der Applikator einheitlich aus einem festen porösen Material hergestellt werden. Ein Luft-Volumenstrom ist groß, da im gesamten Applikatorkörper Luftkanäle gebildet sind. Auch ein innerer Hohlraum des Applikators kann einen Luftkanal bilden.

Die elastische Sterilhülle ist an der Außenoberfläche des Applikators angeordnet. Damit bilden der Applikator und die Sterilhülle ein System, das den Applikator und die Sterilhülle umfasst.

Eine Sterilhülle ist aus einem sterilen elastischen strahlungsdurchlässigen Werkstoff ausgeführt und kann über die Außenoberfläche des Applikators gestülpt oder gezogen werden. Die Sterilhülle ist biokompatibel und für den direkten Gewebekontakt geeignet. Wenn an dem proximalen Ende des Applikators die Luft abgesaugt wird, kann durch die feste poröse Oberflächenstruktur des Applikators die Luft von jedem Oberflächenbereich der Außenoberfläche zu dem proximalen Ende des Applikators fließen. Dadurch wird zwischen der Sterilhülle und der Außenoberfläche des Applikators ein Vakuum ausgebildet werden, so dass die Sterilhülle formschlüssig an dem Applikator fixiert ist und keine Luftblasen zwischen der Sterilhülle und der Außenoberfläche des Applikators verbleiben. Dadurch können während der Behandlung Abweichungen von der vorgegebenen Bestrahlungsgeometrie vermieden werden, so dass sehr präzise Vorhersagen und Vorgaben der auf das zu behandelnde Körpergewebe einwirkenden Strahlendosis möglich sind. Dabei kann der Applikator auch Hinterschneidungen aufweisen. Durch die Ansaugung, bzw. Vakuumbildung, wird die Sterilhülle zuverlässig und formschlüssig an die Applikatorgeometrie der Außenoberfläche gezogen.

Der Applikator kann eine beliebige Außenkontur aufweisen, wobei die Kontur keine spitzen oder scharfen Konturbereiche aufweist, um eine gleichmäßige Bestrahlung eines Körpergewebes zu bewirken und um die an dem Applikator angeordnete Sterilhülle nicht zu beschädigen.

Der Applikator hat dabei eine formgebende Funktion, die Sterilhülle bildet die Sterilbarriere zwischen dem Applikator und dem Körpergewebe. Insbesondere ist der Applikator starr ausgebildet und weißt eine formstabile Außenoberfläche auf. Die Sterilhülle kann als günstiger Einwegartikel ausgeführt werden und kann nach dem Einsatz entsorgt werden. Der Applikator braucht nicht sterilisiert zu werden und kann beliebig oft eingesetzt werden. Die Temperaturanforderungen an den Applikator sind somit geringer.

In einer Ausgestaltung der Erfindung weist die Sterilhülle eine Wandstärke zwischen 0,05 mm und 1 mm auf.

Damit kann eine Sterilhülle elastisch und flexibel ausgebildet werden.

In einer Ausgestaltung der Erfindung weisen die Öffnungen oder Poren der porösen Struktur eine größte Abmessung auf, die kleiner oder gleich der Wandstärke der Sterilhülle ist.

Damit werden größere Einbuchtungen der Sterilhülle beim Ansaugen der Sterilhülle auf dem Applikator zuverlässig verhindert.

In einer Ausgestaltung der Erfindung weist eine Kanalstruktur an der Außenoberfläche des Applikators eine Kanalbreite auf, die kleiner oder gleich der Wandstärke der Sterilhülle ist.

Damit wird werden größere Einbuchtungen der Sterilhülle beim Ansaugen der Sterilhülle auf dem Applikator zuverlässig verhindert.

In einer Ausgestaltung der Erfindung weisen Öffnungen oder Kanäle einer porösen Struktur an der Außenoberfläche des Applikators eine größte Abmessung auf, die in einem Bereich zwischen 0,1mm und 1mm ausgebildet ist.

Damit wird werden größere Einbuchtungen der Sterilhülle beim Ansaugen der Sterilhülle auf dem Applikator zuverlässig verhindert.

In einer Ausgestaltung der Erfindung ist an einer Öffnung der Sterilhülle ein Stützring angeordnet. Vorteilhaft kann die Sterilhülle leicht über den Applikator gestülpt werden. Der Stützring bildet zudem eine Befestigungsmöglichkeit an der Bestrahlungsvorrichtung. Der Stützring bildet eine weitere Abdichtung relativ zu der Bestrahlungsvorrichtung. Die Sterilhülle bildet mit der Bestrahlungsvorrichtung eine durchgehende Sterilbarriere.

In einer Ausgestaltung der Erfindung umfasst eine Röntgenbestrahlungsvorrichtung ein Applikator-System, wobei die Röntgenbestrahlungsvorrichtung eine Vakuumpumpe aufweist.

Eine Röntgenbestrahlungsvorrichtung ist eine Bestrahlungsvorrichtung. Eine Vakuumpumpe stellt die Saugleistung für das Ansaugen der Sterilhülle an dem Applikator bereit. Damit kann die Bestrahlungsvorrichtung mit einem Applikator und einer Sterilhülle komfortabel und schnell für einen Einsatz eingerichtet werden. Eine Vakuumpumpe kann vorteilhaft in einen Saug- und Druckbetrieb eingesetzt werden.

An Applikator können Maskierungen angebracht werden. Maskierungen können individuell unsteril unterhalb der Sterilhülle angeordnet werden, um eine gezielte anisotrope Strahlendosisabgabe zu ermöglichen. Dies kann Behandlungszeiten reduzieren und die Strahlenbelastung gezielter steuern und die Strahlenbelastung anderer Gewebebereiche reduzieren.

An dem Applikator können Abschirmungen angeordne werden.

Abschirmungen reduzieren vorteilhaft Nebenwirkungen für einen Patienten, da Gewebebereiche vor Strahlung geschützt werden können. Abschirmungen brauchen nicht steril zu sein, da diese unterhalb der Sterilhülle angeordnet werden können.

Die Abschirmungen können an dem Applikator im Schaftbereich angeordnet werden.

Vorteilhaft können mögliche strahlungsbedingte Nebenwirkungen für einen Patienten in diesem Bereich reduziert werden. Die Abschirmungen können unterhalb der Sterilhülle angeordnet werden und brauchen deshalb nicht steril zu sein.

Der Applikator ist durch einen 3D-Druck herstellbar. Vorteilhaft sind alle möglichen Konturen und Geometrien des Applikators herstellbar. Kanäle können vorteilhaft in den Applikatorkörper integriert werden. Die Kanäle können an verschiedenen Stellen verschiedene Formen und Querschnitte aufweisen. Spezial-Applikatoren sind für Einzelfälle schnell und kostengünstig realisierbar.

In dem Applikator können zusätzliche Kühlkanäle angeordnet werden.

Dies ist vorteilhaft für den Einsatz in einem Gewebebereich und verringert die Gefahr von Temperaturschädigungen des Gewebes.

Ein Verfahren zum Aufbringen einer Sterilhülle auf einen Applikator, der an einer Röntgenbestrahlungsvorrichtung mit einer Vakuumpumpe angeordnet ist, umfasst die Schritte:
- Überziehen der Sterilhülle über den Applikator,
- Ansteuern der Vakuumpumpe in einem Pumpbetrieb, sodass Luft über die Außenoberfläche des Applikators in die Sterilhülle hineinströmt,
- Positionieren der Sterilhülle an dem Applikator,
- Ansteuern der Vakuumpumpe in einem Vakuumbetrieb, sodass Luft über die Außenoberfläche des Applikators aus der Sterilhülle abgesaugt wird, sodass die Sterilhülle fest an der Außenoberfläche anliegt.

Dieses Verfahren erleichtert das Anbringen einer Sterilhülle auf einem Applikator indem zunächst Luft in die Sterilhülle geblasen wird, so dass die Sterilhülle ballonartig aufgeblasen wird. In dieser Situation entsteht zwischen dem Applikator und der Sterilhülle kaum Reibung, so dass Faltenbildung und Überlappungen reduziert werden und die Sterilhülle einfach positioniert werden kann. Danach wechselt die Vakuum-Pumpe in einen Saugbetrieb, sodass die Luft aus der Sterilhülle abgesaugt wird und diese eng und fest an der Außenoberfläche des Applikators anliegt. Die Sterilhülle kann gleichmäßig an der Außeroberfläche anliegen, da keine Bereiche der Sterilhülle stark gedehnt oder gestaucht werden. Die Vakuumpumpe ist ausgebildet, wechselweise in einem Druckbetrieb oder in einem Vakuum-Betrieb eingesetzt zu werden.

Beim Entfernen einer Sterilhülle von dem Applikator kann die Vakuumpumpe wieder in einem Pumpbetrieb angesteuert werden, sodass Luft über die Außenoberfläche des Applikators in die Sterilhülle einströmt und diese einfach von dem Applikator abgezogen werden und entfernt werden kann.

Weitere Vorteile und Merkmale der Erfindung werden in Bezug auf die nachfolgenden Zeichnungen erklärt.

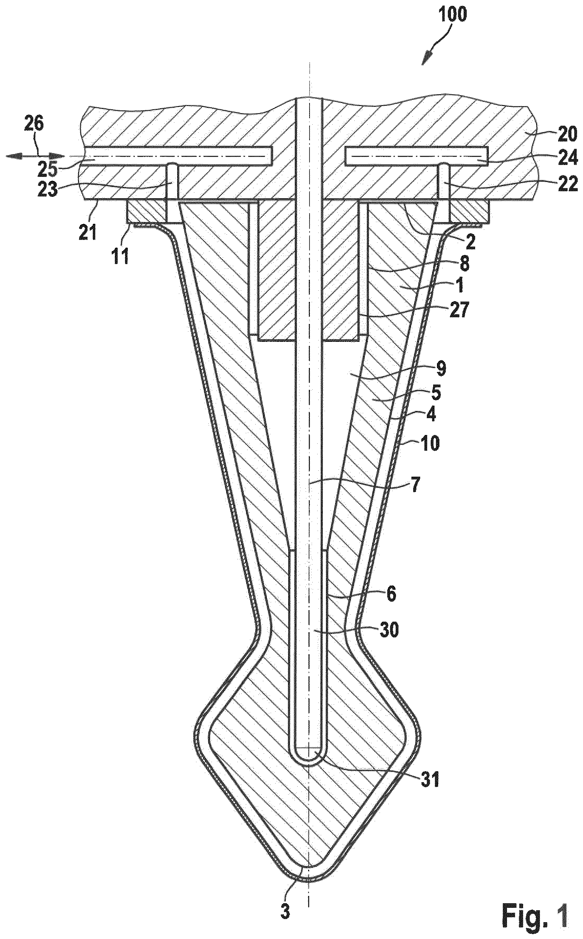

Die Figur 1 zeigt ein Ausführungsbeispiel eines Applikators für die intraoperative Strahlentherapie mit einer hochelastischen Sterilhülle in einer Schnittdarstellung.

Ein Strahlentherapiesystem 100 umfasst eine Röntgenbestrahlungsvorrichtung 20 mit einem Aufnahmezapfen 27. In dem Aufnahmezapfen 27 ist ein Rohr 30 angeordnet. An dem distalen Ende des Rohres 30 ist ein nicht dargestellter Verschluss angeordnet. Die Innenfläche des Verschlusses ist mit einem Strahlungsmedium 31 belegt, die als Gold-Target ausgeführt ist. Die Röntgenbestrahlungsvorrichtung 20 umfasst eine nicht dargestellte Elektronenstrahlquelle, die einen Elektronenstrahl entlang einer Mittelachse 7 emittiert. Der Elektronenstrahl trifft auf das Strahlungsmedium 31, das Gold-Target, mit einer kinetischen Energie von maximal 100 keV, in einer Ausführungsform 50 keV, so dass mit hoher Effizienz Röntgenstrahlung am Ort des Strahlungsmediums 31 erzeugt wird. Das distale Ende des Rohres 30 bildet somit eine niederenergetische Röntgenquelle. Das Rohr 30 kann aus rostfreiem Stahl hergestellt sein.

Der auf das distale Ende des Rohres 30 auftreffende Elektronenstrahl erzeugt die Röntgenstrahlung isotrop, d. h. mit gleicher Intensität in sämtliche Raumrichtungen. Dabei ist die Strahlungsintensität am distalen Ende des Rohres 30 am höchsten und nimmt mit zunehmendem Abstand vom distalen Ende des Rohres 30 ab.

An dem Aufnahmezapfen 27 ist ein Applikator 1 mit einem Applikatorkörper 5 angeordnet. Der Applikator 1 ist mit einer Aufnahmevorrichtung 8, die durch eine Führungsöffnung gebildet ist, auf den Aufnahmezapfen 27 gestülpt und liegt mit seinem proximalen Ende 2 an der Anlagefläche 21 der Röntgenbestrahlungsvorrichtung 20 an. Eine innere Aussparung 9, bzw. ein innerer Hohlraum des Applikators 1 ist derart ausgebildet, dass das Rohr 30 der Röntgenbestrahlungsvorrichtung 20 darin aufgenommen werden kann. Die innere Aussparung 9 hat eine zylindrisch ausgebildete Führungsöffnung am distalen Ende 2 des Applikators 1. Im weiteren Verlauf ist die innere Aussparung 9 kegelförmig ausgebildet und mündet in einen zylindrischen Bereich 6, in dem das distale Ende des Rohres 30 angeordnet ist. Die Größe und Gestalt des Applikators 1 definiert den Abstand zwischen dem Strahlungsmedium 31, das eine Strahlungsquelle am distalen Ende der Rohres 30 bildet und einem zu bestrahlenden Gewebe. Durch den Applikator wird der Abstand des Gewebes zu dem Strahlungsmedium 31 definiert, so dass eine gewünschte und/oder gleichmäßige Bestrahlungsdosis das Gewebe erreicht.

Der Applikator 1 ist rotationssymmetrisch um die Mittelachse 7 ausgebildet. Das Strahlungsmedium 31 ist auf der Mittelachse 7 in einem zentralen Bereich des Applikators 1 angeordnet, der vorgesehen ist, in eine Körperöffnung eingebracht zu werden. Ein distales Ende 3 des Applikators kann bis zu einer von der Anwendung abhängigen Tiefe in einen nicht darstellten Gewebebereich einer zu behandelnden Person eingeführt werden.

Der Applikator hat eine Außenoberfläche 4. Die Außenoberfläche 4 ist durch die Oberfläche am Umfang und an einem distalen Ende 3 definiert. Über der Außenoberfläche 4 ist eine Sterilhülle 10 angeordnet. Die Sterilhülle 10 besteht aus einem sterilen elastischen strahlungsdurchlässigen Werkstoff und ist für den direkten Gewebekontakt geeignet. Die Sterilhülle 10 und der Applikator 1 bilden zwei trennbare Teile.

An der Öffnungsseite der Sterilhülle 10 ist ein Stützring 11 angeordnet. Der Stützring 11 bildet eine Abdichtung zwischen der Sterilhülle 10 und der Anlagefläche 21 der Röntgenbestrahlungsvorrichtung 20. Damit ist der Applikator 1 vollständig von der Anlagefläche 21 und der Sterilhülle 10 mit dem Stützring 11 umschlossen. Der Applikator 1 hat dabei eine formgebende Funktion und ist starr ausgebildet, die Sterilhülle 10 bildet eine sterile Barriere zwischen dem Applikator 1 und einem Körpergewebe.

Durch die starre Form der Außenoberfläche 4 des Applikators 1 ist gewährleistet, dass die Außenoberfläche dauerhaft in einem vorgegebenen Abstand zum Strahlungsmedium 31 angeordnet ist und auch im Übrigen eine feste Geometrie aufweist. Dies hat wiederum zur Folge, dass das die Bestrahlungsgeometrie fest vorgegeben ist und das zur Außenoberfläche 4 benachbarte Körpergewebe genau der vor der Behandlung gemessenen oder berechneten Strahlendosis ausgesetzt ist und die Strahlenbehandlung somit sehr präzise durchgeführt werden kann.

Die Außenoberfläche 4 des Applikators 1 bildet eine feste poröse Oberflächenstruktur mit Löchern, Hohlräumen und/oder Kanälen. Die Löcher, Hohlräume und/oder Kanäle bilden ein verzweigtes System aus vielen Luftkanälen, die luftleitend mit dem proximalen Ende 2 des Applikators 1 verbunden sind. Dadurch kann ein Luftstrom von jedem Bereich der Außenoberfläche 4 zu dem proximalen Ende Applikators gebildet werden. Der Luftstrom kann in beide Richtungen erfolgen, entweder von der Außenoberfläche 4 zu dem proximalen Ende 2 oder umgekehrt von dem proximalen Ende 2 zu der Außenoberfläche 4.

An der Anlagefläche 21 der Röntgenbestrahlungsvorrichtung 20 sind ein erster Luftkanal 22 und ein zweiter Luftkanal 23 angeordnet. Der erste Luftkanal 22 ist luftleitend mit einem dritten Luftkanal 24 verbunden. Der zweite Luftkanal 23 ist luftleitend mit einem vierten Luftkanal 25 verbunden. Der dritte Luftkanal 24 ist über eine in der Schnittdarstellung nicht sichtbare Verbindung an den vierten Luftkanal 25 luftleitend angeschlossen. Der vierte Luftkanal 25 ist an eine nicht dargestellte Vakuumpumpe angeschlossen. Der Luftstrom durch den vierten Luftkanal ist schematisch durch einen Doppelpfeil 26 angedeutet.

Die feste poröse Struktur an der Außenoberfläche 4 des Applikators 1 ist luftleitend mit dem proximalen Ende 2 des Applikators 1 verbunden. Die Vakuumpumpe, der erste Luftkanal 22, der zweite Luftkanal 23, der dritte Luftkanal 24 und der vierte Luftkanal 25 bilden somit ein Luft-Absaugsystem oder ein Luft-Ausblassystem für den Applikator 1.

Wenn an dem proximalen Ende des Applikators die Luft durch dieses Absaugsystem abgesaugt wird, kann durch die poröse Oberflächenstruktur des Applikators 1 die Luft von jedem Oberflächenbereich der Außenoberfläche 4 zu dem proximalen Ende 2 des Applikators 1 strömen. Dadurch wird zwischen der Sterilhülle 10 und der Außenoberfläche 4 des Applikators 1 ein Vakuum ausgebildet, so dass die Sterilhülle 10 formschlüssig an dem Applikator 1 fixiert ist. Dabei kann der Applikator 1 auch Hinterschneidungen aufweisen. Durch die Ansaugung wird die Sterilhülle 10 zuverlässig und formschlüssig an die äußere Geometrie des Applikators 1 gezogen. Die poröse Oberflächenstruktur des Applikators 1 ist so ausgebildet, dass keine isolierten Oberflächenbereiche vorhanden sind, aus denen die Luft nicht abgesaugt wird um demgemäß dort Luftblasen zurückbleiben. Die Ansaugung der Sterilhülle 10 durch die poröse Struktur der Außenoberfläche 4 trägt somit ebenfalls dazu bei, dass das Körpergewebe einer genau definierten Strahlendosis ausgesetzt werden kann. Würden Luftblasen zwischen der Sterilhülle 10 und der Außenoberfläche 4 verbleiben, so hätte das einen Einfluss auf die Bestrahlungsgeometrie, insbesondere auf den Abstand zwischen dem Strahlungsmedium 31 und dem Körpergewebe und damit auf die Strahlendosis im Bereich des Körpergewebes. Durch die Luftblasen könnte es somit auch im Falle einer starren Außenoberfläche 4 zu Abweichungen von der vorhergesagten Strahlendosis kommen.

Der Saugleistung oder Druckleistung der Vakuumpumpe kann variabel eingestellt und geregelt werden. In einer Ausführungsform kann in einem Luftkanal ein nicht dargestellter Drucksensor angeordnet sein. Es kann eine Saugleistung oder ein Druck p0 definiert sein, bis zu dem eine leichte Ansaugwirkung für die Sterilhülle 10 bewirkt wird, so dass die Sterilhülle 10 auf dem Applikator 1 formschlüssig ausgerichtet werden kann, beispielsweise um Faltenbildung oder Überlappungen zu verhindern. Die Vakuumpumpe kann auch nach Erreichen des Druckes p0 abgeschaltet werden.

Wenn die Sterilhülle 10 optimal ausgerichtet ist, kann der Druck weiter verringert werden, um ein definiertes Anliegen der Sterilhülle 10 an dem Applikator 1 zuverlässig zu gewährleisten. Danach kann die Saugleistung der Vakuumpumpe abgeschaltet oder auf einen Wert reduziert werden, der mögliche Luftleckströme ausgleicht, so dass das dichte formschlüssige Anliegen der Sterilhülle 10 während des gesamten Einsatzes in einem Gewebebereich zuverlässig gewährleistet ist.

Es kann auch ein Druck p1 definiert sein, bei dem die Vakuumpumpe in einen Druckbetrieb geschaltet wird, so das durch Einblasen von Luft in den Applikator 1 eine leichte Druckwirkung auf die Sterilhülle 10 bewirkt wird. Die Sterilhülle 10 wird leicht aufgeblasen und kann somit bei besonders geringer Reibung auf dem Applikator 1 formschlüssig ausgerichtet werden. Danach kann die Vakuumpumpe in einen Saugbetrieb umgeschaltet werden. Der Druck kann verringert werden, um ein Ansaugen und ein definiertes Anliegen der Sterilhülle 10 an dem Applikator 1 zuverlässig zu gewährleisten. Danach kann die Saugleistung der Vakuumpumpe abgeschaltet oder auf einen Wert reduziert werden, der mögliche Luftleckströme ausgleicht.

In einer Ausführungsform ist die Außenoberfläche 4 bis zu einer bestimmten Tiefe durch eine feste poröse Struktur mit feinen Löchern, Hohlräumen und/oder Kanälen ausgebildet. Die Tiefe kann in einem Bereich zwischen 1 mm und 10 mm ausgebildet sein.

In einer Ausführungsform besteht der gesamte Applikatorkörper 5 aus einer festen porösen Struktur mit feinen Löchern, Hohlräumen und/oder Kanälen.

In einer Ausführungsform sind in dem Körper des Applikators 1 ein oder mehrere zusätzliche Luftleitungskanäle mit einem Querschnitt größer als 1mm² ausgebildet, die von dem proximalen Ende 2 zu einer oder mehreren Stellen der porösen Struktur der Außenoberfläche 4 geführt sind.

### Bezugszeichenliste

- 100: Strahlentherapie-System

- 1: Applikator
- 2: Proximales Ende des Applikators
- 3: Distales Ende des Applikators
- 4: Außenoberfläche
- 5: Applikatorkörper
- 6: Zylindrischer Bereich der inneren Aussparung
- 7: Mittelachse
- 8: Aufnahmevorrichtung
- 9: Innere Aussparung

- 10: Sterilhülle
- 11: Stützring

- 20: Röntgenbestrahlungsvorrichtung
- 21: Anlagefläche
- 22: Erster Luftkanal
- 23: Zweiter Luftkanal
- 24: Dritter Luftkanal
- 25: Vierter Luftkanal
- 26: Doppelpfeil
- 27: Aufnahmezapfen

- 30: Rohr
- 31: Strahlungsmedium

## Patentansprüche

1. Applikator-System umfassend einen Applikator (1) für die intraoperative Strahlentherapie mit niederenergetischer Röntgenstrahlung und eine elastische Sterilhülle (10), wobei der Applikator (1)
- einen Applikatorkörper (5),
- eine Außenoberfläche (4) mit einer Umfangsaußenfläche und einem distalen Ende,
- eine Aufnahmevorrichtung (8) an einem proximalen Ende (2), mit der der Applikator (1) an einer Röntgenbestrahlungsvorrichtung (20) befestigbar ist, und
- eine innere Aussparung (9) mit einer Öffnung an dem proximalen Ende (2), in die eine Röntgenstrahlungsquelle (30, 31) einführbar ist,
umfasst und
wobei der Applikator (1) an seiner Außenoberfläche (4) eine feste poröse Struktur aufweist, wobei die feste poröse Struktur eine luftdurchlässige zusammenhängende Kanalstruktur bildet, die luftleitend mit dem proximalen Ende (2) des Applikators (1) verbunden ist und der Außenoberfläche (4) des Applikators (1) eine starre Form verleiht,
wobei die Sterilhülle an der Außenoberfläche des Applikators anordenbar ist.

2. Applikator-System nach Anspruch 1,
wobei die feste poröse Struktur durch eine feine Kanalstruktur gebildet ist, die an der Außenoberfläche (4) des Applikators (1) angeordnet ist.

3. Applikator-System nach einem der vorherigen Ansprüche,
wobei innerhalb des Applikatorkörpers (5) Kanäle angeordnet sind, die von dem proximalen Ende (2) zu der luftdurchlässigen Außenoberfläche (4) geführt sind, so dass die luftdurchlässige Außenoberfläche (4) des Applikators (1) luftleitend mit dem proximalen Ende (2) verbunden ist.

4. Applikator-System nach einem der vorherigen Ansprüche,
wobei der gesamte Applikatorkörper (5) eine feste poröse Struktur aufweist.

5. Applikator-System nach einem der vorherigen Ansprüche,
wobei die Sterilhülle (10) eine Wandstärke zwischen 0,05 mm und 1 mm aufweist.

6. Applikator-System nach einem der vorherigen Ansprüche,
wobei die Öffnungen oder Poren der porösen Struktur eine größte Abmessung aufweisen, die kleiner oder gleich der Wandstärke der Sterilhülle (10) ist.

7. Applikator-System nach Anspruch 5, rückbezogen auf Anspruch 2,
wobei eine Kanalstruktur an der Außenoberfläche (4) des Applikators (1) eine Kanalbreite aufweist, die kleiner oder gleich der Wandstärke der Sterilhülle (10) ist.

8. Applikator-System nach einem der Ansprüche 1 bis 4,
wobei Öffnungen oder Kanäle einer porösen Struktur an der Außenoberfläche (4) des Applikators (1) eine größte Abmessung aufweisen, die in einem Bereich zwischen 0,1 mm und 1 mm ausgebildet ist.

9. Applikator-System nach einem der vorherigen Ansprüche,
wobei an der Öffnung der Sterilhülle (10) ein Stützring (11) angeordnet ist.

10. Röntgenbestrahlungsvorrichtung (20) mit einem Applikator-System nach einem der
vorherigen Ansprüche, wobei die Röntgenbestrahlungsvorrichtung (20) eine Vakuumpumpe aufweist.

11. Verfahren zum Aufbringen einer Sterilhülle (10) auf einen Applikator (1), in einem Applikator-System nach einem der Ansprüche 1 bis 9, wobei der Applikator an einer
Röntgenbestrahlungsvorrichtung (20) mit einer Vakuumpumpe angeordnet ist, umfassend die Schritte:
Überziehen der Sterilhülle (10) über den Applikator (1),
Ansteuern der Vakuumpumpe in einem Pumpbetrieb, sodass Luft über die Außenoberfläche (4) des Applikators (1) in die Sterilhülle (10) hineinströmt,
Positionieren der Sterilhülle (10) an dem Applikator,
Ansteuern der Vakuumpumpe in einem Vakuumbetrieb, sodass Luft über die Außenoberfläche (4) des Applikators (1) aus der Sterilhülle (10) abgesaugt wird, sodass die Sterilhülle fest an der Außenoberfläche (4) anliegt.

## Claims

1. Applicator system comprising an applicator (1), for intraoperative radiotherapy with low-energy X-ray radiation, and an elastic sterile sleeve (10), wherein the applicator (1) comprises
- an applicator body (5),
- an outer surface (4) with a circumferential outer face and with a distal end,
- a receiving device (8) which is arranged at a proximal end (2) and with which the applicator (1) can be secured to an X-ray irradiation device (20), and
- an inner recess (9) which has an opening at the proximal end (2) and into which an X-ray radiation source (30, 31) is insertable, and
wherein the applicator (1) has a solid porous structure on its outer surface (4), wherein the solid porous structure forms a continuous air-permeable channel structure which is connected in an air-conducting manner to the proximal end (2) of the applicator (1) and gives the outer surface (4) of the applicator (1) a rigid form, wherein the sterile sleeve can be arranged on the outer surface of the applicator.

2. Applicator system according to Claim 1, wherein the solid porous structure is formed by a fine channel structure arranged on the outer surface (4) of the applicator (1).

3. Applicator system according to one of the preceding claims, wherein channels running from the proximal end (2) to the air-permeable outer surface (4) are arranged inside the applicator body (5), such that the air-permeable outer surface (4) of the applicator (1) is connected in an air-conducting manner to the proximal end (2).

4. Applicator system according to one of the preceding claims, wherein the entire applicator body (5) has a solid porous structure.

5. Applicator system according to one of the preceding claims, wherein the sterile sleeve (10) has a wall thickness of between 0.05 mm and 1 mm.

6. Applicator system according to one of the preceding claims, wherein the openings or pores of the porous structure have a greatest dimension that is smaller than or equal to the wall thickness of the sterile sleeve (10).

7. Applicator system according to Claim 5, referred back to Claim 2, wherein a channel structure on the outer surface (4) of the applicator (1) has a channel width that is smaller than or equal to the wall thickness of the sterile sleeve (10).

8. Applicator system according to one of Claims 1 to 4, wherein openings or channels of a porous structure on the outer surface (4) of the applicator (1) have a greatest dimension that is in a range of between 0.1 mm and 1 mm.

9. Applicator system according to one of the preceding claims, wherein a support ring (11) is arranged at the opening of the sterile sleeve (10).

10. X-ray irradiation device (20) with an applicator system according to one of the preceding claims, wherein the X-ray irradiation device (20) has a vacuum pump.

11. Method for applying a sterile sleeve (10) to an applicator (1) in an applicator system according to one of Claims 1 to 9, wherein the applicator is arranged on an X-ray irradiation device (20) with a vacuum pump, said method comprising the steps of:
pulling the sterile sleeve (10) over the applicator (1),
controlling the vacuum pump in a pump mode, such that air flows through the outer surface (4) of the applicator (1) into the sterile sleeve (10),
positioning the sterile sleeve (10) on the applicator,
controlling the vacuum pump in a vacuum mode, such that air is aspirated through the outer surface (4) of the applicator (1) from the sterile sleeve (10), such that the sterile sleeve bears firmly on the outer surface (4).

## Revendications

1. Système d'applicateur comprenant un applicateur (1) pour la radiothérapie peropératoire avec des rayons X de faible énergie et une enveloppe stérile élastique (10), l'applicateur (1) comprenant
- un corps d'applicateur (5),
- une surface extérieure (4) avec une face extérieure périphérique et une extrémité distale,
- un dispositif de réception (8) à une extrémité proximale (2), avec lequel l'applicateur (1) peut être fixé à un dispositif d'exposition aux rayons X (20), et
- un évidement intérieur (9) avec une ouverture à l'extrémité proximale (2), dans lequel une source de rayons X (30, 31) peut être insérée,
et
l'applicateur (1) présentant une structure poreuse solide sur sa surface extérieure (4), la structure poreuse solide formant une structure de canaux contigus perméable à l'air, qui est reliée à l'extrémité proximale (2) de l'applicateur (1) de manière à conduire l'air et confère une forme rigide à la surface extérieure (4) de l'applicateur (1),
l'enveloppe stérile pouvant être agencée sur la surface extérieure de l'applicateur.

2. Système d'applicateur selon la revendication 1, la structure poreuse solide étant formée par une structure de canaux fins qui est agencée sur la surface extérieure (4) de l'applicateur (1).

3. Système d'applicateur selon l'une quelconque des revendications précédentes, des canaux étant agencés à l'intérieur du corps de l'applicateur (5), qui sont acheminés de l'extrémité proximale (2) à la surface extérieure perméable à l'air (4), de telle sorte que la surface extérieure perméable à l'air (4) de l'applicateur (1) est reliée à l'extrémité proximale (2) de manière à conduire l'air.

4. Système d'applicateur selon l'une quelconque des revendications précédentes, l'ensemble du corps d'applicateur (5) présentant une structure poreuse solide.

5. Système d'applicateur selon l'une quelconque des revendications précédentes, l'enveloppe stérile (10) présentant une épaisseur de paroi comprise entre 0,05 mm et 1 mm.

6. Système d'applicateur selon l'une quelconque des revendications précédentes, les ouvertures ou pores de la structure poreuse présentant une dimension maximale qui est inférieure ou égale à l'épaisseur de paroi de l'enveloppe stérile (10).

7. Système d'applicateur selon la revendication 5, se référant à la revendication 2, une structure de canaux sur la surface extérieure (4) de l'applicateur (1) présentant une largeur de canaux qui est inférieure ou égale à l'épaisseur de paroi de l'enveloppe stérile (10).

8. Système d'applicateur selon l'une quelconque des revendications 1 à 4, des ouvertures ou canaux d'une structure poreuse sur la surface extérieure (4) de l'applicateur (1) présentant une dimension maximale qui est réalisée dans une plage comprise entre 0,1 mm et 1 mm.

9. Système d'applicateur selon l'une quelconque des revendications précédentes, un anneau de support (11) étant agencé sur l'ouverture de l'enveloppe stérile (10).

10. Dispositif d'exposition aux rayons X (20) avec un système d'applicateur selon l'une quelconque des revendications précédentes, le dispositif d'exposition aux rayons X (20) présentant une pompe à vide.

11. Procédé d'application d'une enveloppe stérile (10) sur un applicateur (1), dans un système d'applicateur selon l'une quelconque des revendications 1 à 9, l'applicateur étant agencé sur un dispositif d'exposition aux rayons X (20) avec une pompe à vide, comprenant les étapes suivantes :
le revêtement de l'enveloppe stérile (10) sur l'applicateur (1),
la commande de la pompe à vide dans un mode de pompage de telle sorte que de l'air s'écoule dans l'enveloppe stérile (10) par l'intermédiaire de la surface extérieure (4) de l'applicateur (1),
le positionnement de l'enveloppe stérile (10) sur l'applicateur,
la commande de la pompe à vide dans un mode de fonctionnement sous vide, de telle sorte que de l'air est aspiré hors de l'enveloppe stérile (10) par l'intermédiaire de la surface extérieure (4) de l'applicateur (1), de telle sorte que l'enveloppe stérile est fermement appliquée contre la surface extérieure (4).
